# EUROPÄISCHE PATENTANMELDUNG

(11) **EP 1 770 024 A2**
(43) Veröffentlichungstag der Anmeldung: **04.04.2007**
(21) Anmeldenummer: 06017403.4
(22) Anmeldetag: 22.08.2006
(51) Int. Cl.: B65D 75/58

(54) **Verpackung**

(30) Priorität: 28.09.2005 DE 202005015085 U
(71) Anmelder: Klocke Verpackungs-Service GmbH, 76356 Weingarten (DE)
(72) Erfinder: Schuehrer, Herbert, 76646 Bruchsal (DE)
(74) Vertreter: Frank, Gerhard

(57) **Zusammenfassung**

Eine Verpackung besteht aus zwei randseitig verbundenen Folien, die mindestens eine Kammer einschließen, in der sich ein mit einem Medium versehener oder zu versehender Träger (3.0) befindet. In einer ersten Folie (2.1) verläuft eine linienförmige Sollbruchstelle (2.1.2) zur Freilegung des Trägers, über die sich der Träger erstreckt, zwischen zweiter Folie (2.2) und Träger (3.0) befindet sich eine weitgehend starre Einlage (1.0), die sich über die ihr gegenüberliegende Sollbruchstelle (2.1.2) zumindest so weit erstreckt, dass beim Aufbrechen der Verpackung der Träger (3.0) aus der Kammer um ein entsprechendes Maß zur Entnahme oder zur Anwendung des Mediums (4.0) herausgezogen wird.

## Beschreibung

Die Erfindung betrifft eine Verpackung aus zwei randseitig verbunden Folien, die mindestens eine Kammer einschließen, in der sich ein mit einem Medium versehener oder zu versehender Träger befindet, wobei in einer ersten Folie eine linienförmige Sollbruchstelle zur Freilegung des Trägers verläuft, über die sich der Träger erstreckt.

### Technischer Hintergrund

In Medizin, Kosmetik oder im chemischen Bereich bedient man sich zum Speichern oder zum Aufbringen von flüssigen und pastösen Medien oder zur Darreichung von Duftproben saugfähiger Träger, z.B. in Form von Schwämmen oder Vliesen. Im Einsatz sind ebenso flexible Träger aus aufnahmefähigem Material wie z.B. Kunststoff, Papier, oder Karton. Diese saugfähigen Träger werden üblicherweise mit dem einzusetzenden Medium getränkt. Der getränkte, saugfähige Träger kann hierbei je nach Anwendungsfall auch als Applikator genutzt werden, um die zu versorgenden Bereiche mit dem Medium zu benetzen.

Wird das aufzutragende Medium aus größeren Gebinden entnommen, so besteht die Gefahr von ungewolltem Kontakt mit dem Medium oder auch dessen Verunreinigung. Um Abhilfe zu schaffen, wurden Packungen für Einmalanwendungen entwickelt, die ein weitgehend hygienisches Arbeiten auch für den Laien ermöglichen.

Ein Beispiel für derartige Anwendungen sind Verpackungen mit einer Sollbruchstelle, welche sowohl das anzuwendende Medium, als auch einen Applikator beinhalten können.

Um Produkt, Anwender und zu benetzende Bereiche optimal zu schützen, ist der Applikator in die Vertiefung einer vorzugsweise thermoplastisch verformten Kunststoff-Folie eingelegt, der Applikator kann dabei mit dem aufzubringenden Medium benetzt sein. Bei anderen Packungsvariationen befindet sich das aufzubringende Medium einer separaten Vertiefung. Die Vertiefungen sind mit einer vorzugsweise durch Siegeln aufgebrachten Deckfolie verschlossen und so gegen äußere Einflüsse geschützt.

Im Bereich der Vertiefung der Verpackung für den Applikator verläuft eine Sollbruchstelle. Ist das aufzubringende Medium in einer separaten Vertiefung gelagert, so wird dieses unmittelbar vor der Anwendung mit dem Applikator zusammengebracht. Um den Applikator zur Anwendung zu bringen, wird die Verpackung durch Knicken der Packung an der Sollbruchstelle geöffnet. Anschließend werden die beiden Verpackungsenden in Richtung der Deckfolie zusammengeführt, so dass der Applikator im Bereich der Sollbruchstelle freigelegt wird. Das aufzutragende Medium kann nun zur Anwendung gelangen.

Nachteil einer solchen Verpackung ist, dass der Applikator nur in geringem Maß über den aufgebrochenen Bereich der Verpackung hinausragt, so dass die Gefahr besteht, dass der äußere Bereich der Verpackung oder die Aufbrechstelle mit dem zu benetzenden Teil in Kontakt kommt. Gerade im medizinischen Bereich, wo eine solche Verpackung beispielsweise zur Wundversorgung angewendet wird, können hier über eine kontaminierte Außenhaut einer solchen Verpackung Keime auf die zu behandelnde Stelle gelangen, oder der zu versorgende Bereich kann durch die Aufbrechkante der Verpakkung in Mitleidenschaft gezogen werden.

### Darstellung der Erfindung

Aufgabe der Erfindung ist es, den Träger bzw. Applikator weiter aus der Verpackung auszutreiben, und zwar ohne Zuhilfenahme äußerer Mittel.

Die Erfindung löst diese Aufgabe mit den Merkmalen des Anspruchs 1.

Die erfindungsgemäße Einlage wirkt als "Austreibeplättchen" zwischen Träger und Deckfolie, welche in ihrer Dimension so gewählt ist, dass sie entweder in ihrer Endkontur über die Aufbruchstelle hinausragt, oder beim Umbiegen an einer Schwächung Teilbereiche der Einlage über die Sollbruchstelle hinausragen. Der Träger oder Applikator ist gezwungen, sich über den Bereich der Einlage zu legen, der die Sollbruchstelle überragt. Dadurch wird der Träger weiter aus der Packung herausgezogen.

Die Anwendung der Verpackung wird dadurch sicherer, die Gefahr einer Kontaminierung durch die Außenhaut der Verpackung, sowie die Verletzungsmöglichkeit durch die Aufbrechkante wird dadurch minimiert.

Weitere Ausgestaltungen sind Unteransprüchen zu entnehmen.

### Kurze Beschreibung der Zeichnungen

Bevorzugte Ausführungsbeispiele werden anhand von Zeichnungen näher erläutert, es zeigen:
- Bild 1: die Einlage in einem ersten Ausführungsbeispiel in der Vorderansicht und Draufsicht,
- Bild 2: einen Schnitt durch eine Verpackung mit Einlage mit Draufsicht,
- Bild 3: eine geöffnete Verpackung mit Einlage,
- Bild 4: die Anwendung der Verpackung nach Bild 3,
- Bild 5: eine Explosionsdarstellung der Verpackung mit Einlage,
- Bild 6 bis Bild 8: ein zweites Ausführungsbeispiel, eine Zweikammerverpackung mit Einlage mit Sollbruchstelle,
- Bild 9: die erste Ausgestaltung der Einlage wie im ersten Ausführungsbeispiel beschrieben,

- Bild 10: die Einlage in einer aktivierten Verpackung, wie im ersten Ausführungsbeispiel beschrieben,
- Bild 11 bis Bild 13: eine zweite Ausgestaltung der Einlage,
- Bild 14 bis Bild 16: eine dritte Ausgestaltung der Einlage.

### Beschreibung der Ausführungsbeispiele

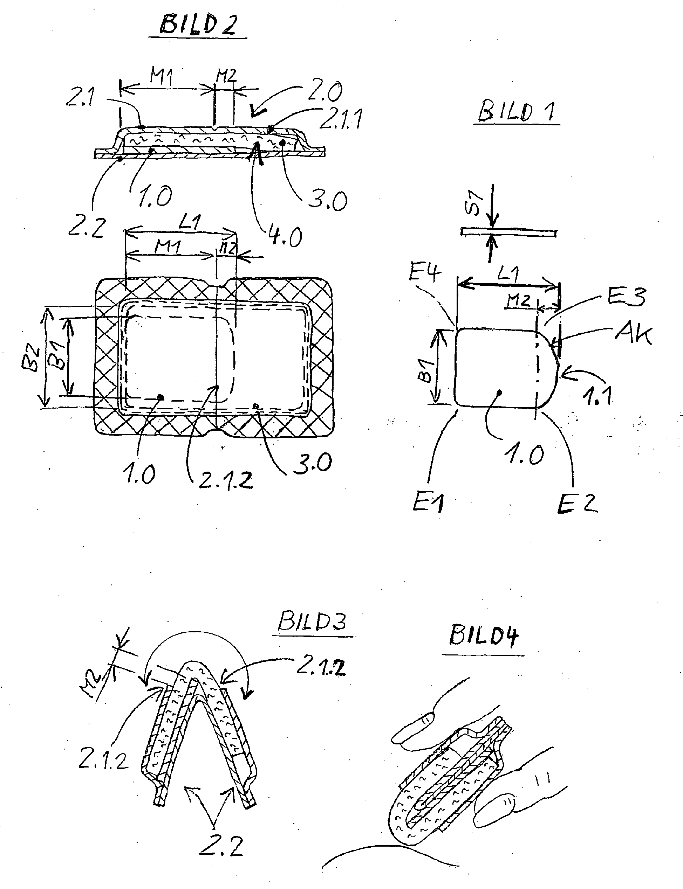

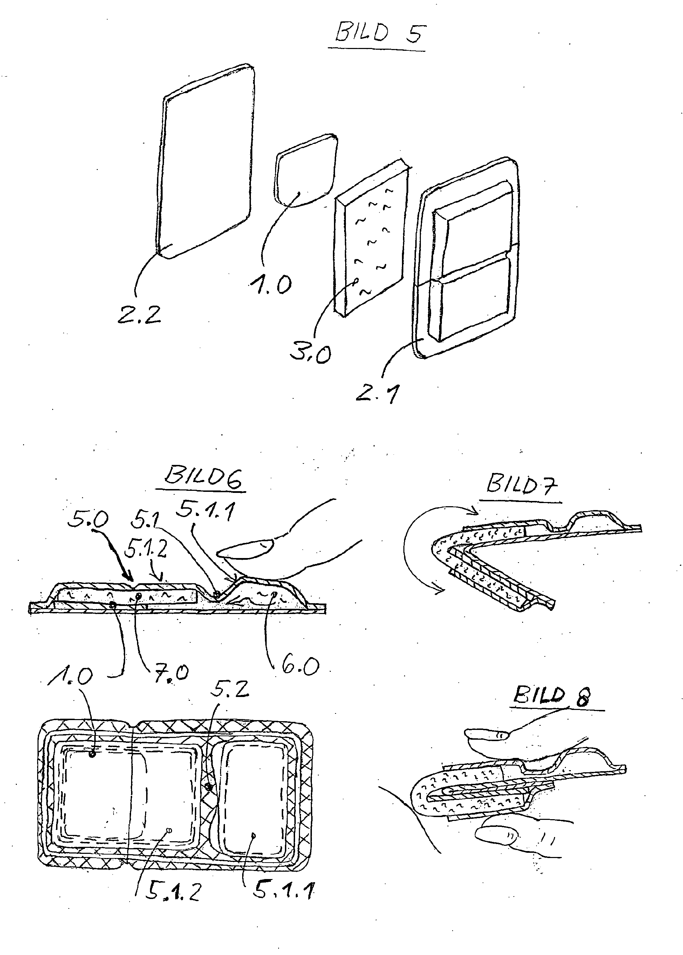

Bild 1 bis Bild 5 zeigen ein erstes Ausführungsbeispiel einer Tiefziehpackung mit Sollbruchstelle und einer Einlage 1.0. Bild 1 zeigt die Einlage 1.0 in der Vorderansicht und in der Draufsicht. Bild 2 zeigt einen Schnitt durch eine Verpackung mit Einlage 1.0 und die Draufsicht. Bild 3 und 4 zeigen eine geöffnete Verpackung mit Einlage und deren Anwendung. Bild 5 zeigt die Explosionsdarstellung der Verpackung mit Einlage 1.0.

Die Einlage 1.0 ist vorzugsweise aus Kunststoff und wird in eine Tiefziehpakkung 2.0, welche aus einer vorzugsweise thermoplastisch verformten Bodenfolie 2.1 mit einer Aufnahmevertiefung 2.1.1 für einen Applikator 3.0 besteht, zwischen Applikator 3.0 und der vorzugsweise durch Siegeln aufgebrachten Deckfolie 2.2 eingelegt. Der Applikator 3.0 ist in diesem Ausführungsbeispiel bereits mit einem aufzutragenden Medium 4.0 benetzt. Die Aufnahmevertiefung 2.1.1 ist mit einer linienförmigen Materialschwächung als Sollbruchstelle 2.1.2 versehen.

Die Einlage 1.0 ist in ihrer Dimension L1 so ausgelegt, dass sie das Maß M1, welches annähernd die Strecke zwischen Beginn der Aufnahmevertiefung 2.1.1 für den Applikator 3.0 und der Sollbruchstelle 2.1.2 beschreibt, um das Maß M2 überragt. Die Breite B1 der Einlage 1.0 ist vorzugsweise geringfügig kleiner gehalten als die Breite B2 des Applikators 3.0. Die Einlage 1.0 kann an ihren Ecken E1,E2,E3,E4 gerundet sein, um die Verletzungsgefahr zu mindern. Eine Außenwölbung 1.1 der Außenkontur AK im Bereich des späteren Zugbereiches erleichtert ein gleichmäßigeres Ausgleiten des Applikators 3.0 aus der Packung 2.0. Bei Aktivierung der Verpackung 2.0 durch Aufbrechen entlang der Sollbruchstelle 2.1.2 und anschließendem Zusammenführen der Verpackungsenden in Richtung der Deckfolie 2.2 (Bild 3), tritt der Überstand M2 der Einlage 1.0 aus der Verpackung 2.0 an der Sollbruchstelle 2.1.2 aus. Der darüber liegende Applikator 3.0 wird gezwungen, sich um diesen Überstand M2 zu legen, wodurch er weiter aus demjenigen Teil der Verpackung ohne Einlage herausgezogen wird. Die Dicke S1 der Einlage 1.0 kann in Abhängigkeit von Material und Herstellungsverfahren gewählt werden und bewegt sich je nach Steifigkeit des Materials vorzugsweise zwischen 0.3 und 3 mm.

Bild 6 bis 8 zeigen ein weiteres Ausführungsbeispiel, eine Tiefziehverpakkung mit Sollbruchstelle 5.0 als 2-Komponentenverpackung, mit einer Einlage 1.0. Die Funktion entspricht der bisherigen Beschreibung. Hier ist das anzuwendende Medium 6.0 in einer separaten Vertiefung 5.1.1 der Bodenfolie 5.1 eingelagert. Durch Zerstörung eines peelfähigen Bereiches 5.2, welcher sich zwischen der Vertiefung 5.1.1 für das anzuwendende Medium 6.0 und der Vertiefung für 5.1.2 für den Applikator 7.0 erstreckt, wird der Applikator 7.0 kurz vor der Anwendung mit dem anzuwendenden Medium benetzt, ohne dass der Inhalt der Packung in Kontakt mit außen liegenden Bereichen gerät.

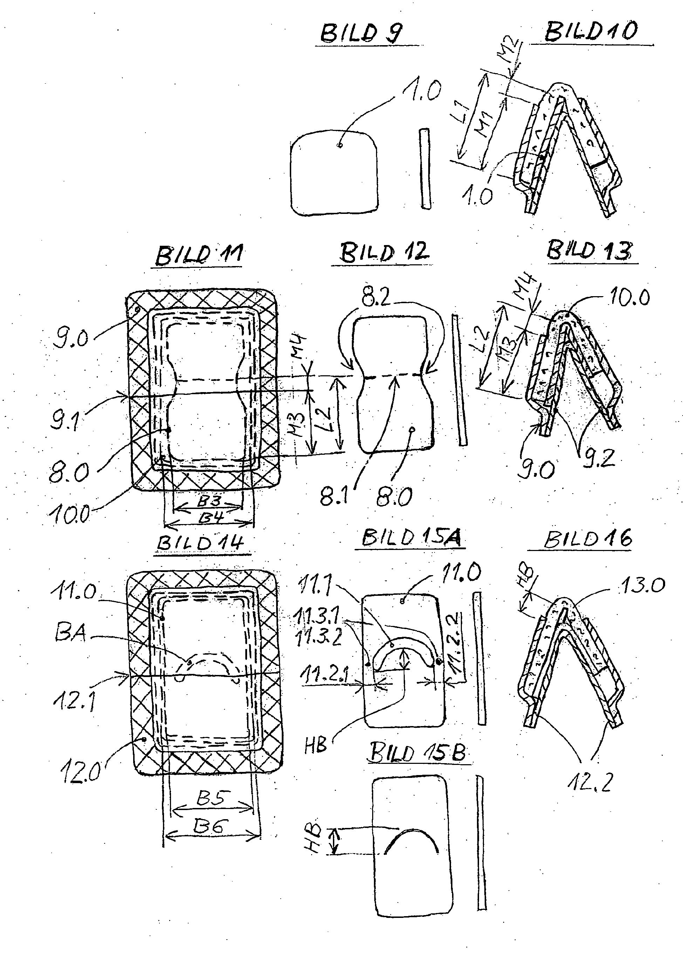

Bild 9 bis Bild 15 zeigen grundsätzliche Ausgestaltungen der Einlage.

Bild 9 zeigt eine Einlage 1.0 wie im ersten Ausführungsbeispiel beschrieben. Bild 10 zeigt die Einlage 1.0 in einer aktivierten Verpackung, wie im ersten Ausführungsbeispiel beschrieben.

Bild 11 bis Bild 13 zeigen in einer zweiten Ausgestaltung eine Einlage 8.0. Bild 11 zeigt eine Verpackung mit Einlage 8.0 in der Draufsicht, Bild 12 zeigt die Einlage 8.0 in Drauf- und Seitenansicht. Bild 13 zeigt die Einlage 8.0 in geöffneter Verpackung.

Die Einlage 8.0 ist für eine Verpackung 9.0 mit Sollbruchstelle 9.1 entsprechend den bisherigen Ausführungsbeispiel vorgesehen, erstreckt sich aber in ihrer Längsdimension über wesentliche Bereiche des Applikators 10.0.

Die Einlage 8.0 ist mit einer Schwächung 8.1, z.B. in Form einer Perforation oder Rillung versehen. Die Schwächung 8.1 ist auf der Einlage 8.0 so angeordnet, dass sie in der Verpackung 9.0 parallel zu deren Sollbruchstelle 9.1 verläuft. Das Maß L2, welches die Position der Schwächung 8.1 bestimmt, ist so dimensioniert, dass dieses bei Einführung der Einlage 8.0 in die Verpakkung 9.0 das Maß M3, welches annähernd die Distanz zwischen Beginn des Aufnahmenapfes für den Applikator 10.0 und der Sollbruchstelle 9.1 beschreibt, um das Maß M4 überragt. Die Breite B3 der Einlage 8.0 ist vorzugsweise geringfügig kleiner als die Breite B4 des Applikators 10.0. Eine Taille 8.2 im Bereich der Schwächung 8.1 vermindert die Gefahr von Verletzungen durch die Einlage 8.0 in diesem Bereich.

Bei Aktivierung der Packung 9.0 durch Aufbrechen entlang der Sollbruchstelle 9.1 und anschließendem Zusammenführen der Packungsenden in Richtung der Deckfolie 9.2 wird die Einlage 8.0 an ihrer Schwächung 8.1 geknickt, wobei der Überstand M4 der Einlage 8.0 aus der Packung 9.0 über die Sollbruchstelle 9.1 hinausragt. Der darüber liegende Applikator 10.0 wird gezwungen, sich um diesen Überstand M4 zu legen, wobei er in einem durch den Überstand M4 definierten Bereich aus der Verpackung ausgeschoben wird.

Bild 14 bis Bild 16 zeigen ein weiteres Ausführungsbeispiel der Verpackung mit einer Einlage 11.0. Die Einlage 11.0 ist für eine Verpackung 12.0 mit Sollbruchstelle 12.1 entsprechend den bisherigen Ausführungsbeispielen vorgesehen. Sie erstreckt sich wie bei ihrer zweiten Ausgestaltung über wesentliche Bereiche des Applikators 13.0.

Die Einlage 11.0 ist mit einer Schwächung 11.1 z.B. in Form einer Stanzung (Bild 15A) oder mit einem Schnitt (Bild 15B) versehen. Die Schwächung 11.1 ist vorzugsweise bogenförmig (andere Formen sind möglich) auf der Einlage 11.0 in der Weise angeordnet, dass der Auslauf des Bogens BA bei Einlage in die Verpackung 12.0 in etwa auf Position der Sollbruchstelle 12.1 der Verpackung 12.0 zu liegen kommt. Die Breite der Stanzung bzw. des Schnittes 11.1 ist so angelegt, dass die Einlage 11.0 an den verbleibenden Stegen 11.2.1 und 11.2.2 die größte Schwächung erfährt. Eine Prägung oder Perforation 11.3.1 und 11.3.2 in diesem Bereich kann die Schwächung noch verstärken. Die Höhe HB des Innenbogens der Stanzung 11.1 ist dabei bestimmend für den Umfang, in dem der Applikator 13.0 im Bereich der Sollbruchstelle 12.1 aus der Verpackung 12.0 ausgetrieben wird. Die Breite B5 der Einlage 11.0 ist vorzugsweise geringfügig kleiner als die Breite B6 des Applikators 13.0.

Bei Aktivierung der Verpackung 12.0 durch Aufbrechen entlang der Sollbruchstelle 12.1 und anschließendem Zusammenführen der Packungsenden in Richtung der Deckfolie 12.2, wird die Einlage 11.0 an ihrer Schwächung 11.2 geknickt, wobei der bogenförmige Bereich HB der Einlage 11.0 über die Sollbruchstelle 12.1 der Packung 12.0 hinausragt. Der darüber liegende Applikator 13.0 wird gezwungen, sich um den bogenförmigen Bereich zu legen und wird somit weiter aus der Packung 12.0 herausgezogen.

Die bisher aufgeführten Beispiele dienen nur zum Veranschaulichen der Funktionsweise der Einlage. Eine derartige Einlage kann bei allen Verpakkungen, bei denen ein Applikator in Teilbereichen ausgetrieben werden soll und die einen ähnlichen Öffnungsmechanismus haben, zum Einsatz kommen.

## Patentansprüche

1. Verpackung aus zwei randseitig verbundenen Folien, die mindestens eine Kammer einschließen, in der sich ein mit einem Medium versehener oder zu versehender Träger befindet, wobei in einer ersten Folie eine linienförmige Sollbruchstelle zur Freilegung des Trägers verläuft, über die sich der Träger erstreckt,
**dadurch gekennzeichnet, dass** sich zwischen zweiter Folie (2.2) und Träger (3.0) eine weitgehend starre Einlage (1.0) befindet, die sich über die ihr gegenüberliegende Sollbruchstelle (2.1.2) zumindest so weit erstreckt, dass beim Aufbrechen der Verpackung der Träger (3.0) des Mediums aus der Kammer um ein entsprechendes Maß zur Entnahme oder zur Anwendung des Mediums (4.0) herausgezogen wird.

2. Verpackung nach Anspruch 1, **dadurch gekennzeichnet, dass** sie eine Tiefziehverpackung ist, bei der die erste Folie mit der Sollbruchstelle die tiefgezogene Bodenfolie (2.1) ist.

3. Verpackung nach Anspruch 1, **dadurch gekennzeichnet, dass** die Breite (B1) der Einlage geringfügig geringer ist als die Breite (B2) des Trägers.

4. Verpackung nach Anspruch 1, **dadurch gekennzeichnet, dass** sich die linienförmige Sollbruchstelle (2.1.2) in der Mitte befindet.

5. Verpackung nach Anspruch 1, **dadurch gekennzeichnet, dass** die Einlage (8.0) eine parallel mit einem Abstand (M4) zur ersten Sollbruchstelle (9.1) der Verpackung verlaufende zweite Sollbruchstelle (8.1) aufweist.

6. Verpackung nach Anspruch 5, **dadurch gekennzeichnet, dass** die Einlage (8.0) in Höhe der zweiten Sollbruchstelle (8.1) seitliche Verjüngungen (8.2) aufweist.

7. Verpackung nach Anspruch 1, **dadurch gekennzeichnet, dass** die Einlage (11.0) eine Schwächung (11.1) in Form einer Stanzung oder eines Schnitts aufweist, die sich bogenförmig von zwei seitlichen Biegestellen (11.3.1; 11.3.2) in Höhe der ersten Sollbruchstelle (12.1) erstreckt, so dass eine Lasche gebildet wird, die beim Aufbrechen den Träger (13.0) teilweise aus der Kammer herauszieht.

8. Verpackung nach Anspruch 1, **dadurch gekennzeichnet, dass** es eine Zwei-Kammerverpackung ist, bei der das aufzutragende Medium (6.0) in einer separaten, zweiten Kammer untergebracht ist, von der es durch Druckausübung in die Kammer mit dem Träger (7.0) beförderbar ist, die die Sollbruchstelle (5.1.3) aufweist.

9. Verpackung nach Anspruch 1, **dadurch gekennzeichnet, dass** der Träger ein Applikator zur Aufbringung des Mediums ist.
